# EUROPEAN PATENT SPECIFICATION

(11) **EP 3 881 789 B1**
(45) Date of publication and mention of the grant of the patent: **28.01.2026**
(21) Application number: 21173383.7
(22) Date of filing: 16.09.2015
(51) Int. Cl.: A61B 18/18

(54) **ACTUATION MECHANISM AND ULTRASONIC SURGICAL INSTRUMENT**
BETÄTIGUNGSMECHANISMUS UND CHIRURGISCHES ULTRASCHALLINSTRUMENT
MÉCANISME D'ACTIONNEMENT ET INSTRUMENT CHIRURGICAL ULTRASONIQUE

(43) Date of publication of application: 22.09.2021
(62) Divisional of application: 15903838.9
(73) Proprietor: Reach Surgical Inc., Tianjin 300462 (CN)
(72) Inventor: Beaupré, Jean, Ohio, 45242 (US)
(74) Representative: Bals & Vogel Patentanwälte PartGmbB

(56) References cited:
- US-A- 5 605 272
- US-A1- 2011 290 853
- US-A1- 2011 295 269
- US-A1- 2014 005 682

## Description

The present application is a divisional application of EP Patent Application No. 15903838.9, filed with the EPO on September 16, 2015, entitled "Actuation Mechanism and Ultrasonic Surgical Instrument".

### Field

The present disclosure relates to the field of medical instrument, and particularly to an actuation mechanism and an ultrasonic surgical instrument.

### Background

Ultrasonic surgical systems are widely used in open and endoscopic surgeries owing to good hemostatic effects and small damages to tissue. A conventional ultrasonic surgical system includes a generator, an ultrasonic transducer, an ultrasonic surgical instrument, a foot switch and other accessories. Typically, the ultrasonic transducer is adapted to convert electrical energy at a resonant frequency that is typically provided by the ultrasonic generator, into mechanical power, for example, into vibration motion, which is further transmitted to the ultrasonic surgical instrument. The mechanical vibratory energy is further amplified and transmitted by the ultrasonic surgical instrument and eventually to be transmitted to the tissue, so as to cutting, coagulation and/or dissection of tissue through intracellular water vaporization, protein hydrogen bonds break, cell disintegration, etc.

The conventional ultrasonic surgical instrument includes a handle portion, an end-effector and a clamping assembly. The handle portion includes a trigger. An operator can hold the handle portion by hand and pull the trigger by fingers to manipulate the clamping assembly disposed on a distal portion of the ultrasonic surgical instrument to open or close, for clamping, grasping, dissecting or holding tissue or vessels, such that the end-effector of the ultrasonic surgical instrument can cut or coagulating the clamped tissue or vessels.

During a surgery, a surgeon usually needs to know the strength of a clamping force applied to tissue through the ultrasonic surgical instrument, since if force is too much, tissue may get damaged, while if force is too little, tissue may not get cut. The conventional ultrasonic surgical instrument includes the handle portion having one trigger. The clamp arm disposed on the distal portion of the instrument is actuated to pivot when the trigger is pulled by the surgeon, so as to clamp the tissue. In this manner, the surgeon may not feel how much force is applied on the tissue very well. In addition, the maximum clamping force is constant when the clamping assembly is actuated to close by pulling the trigger, thus the surgeon cannot adjust the strength of the clamping force based on various conditions of the clamped tissue.

A document (US2011/290853A1) discloses a surgical severing and stapling instrument, suitable for laparoscopic and endoscopic clinical procedures, clamping tissue within an end effector of an elongate channel pivotally opposed by an anvil. Various embodiments are configured to be operably attached to a robotic system to receive actuation/control motions therefrom.

A document (US2014/0005682A1) discloses various embodiments of surgical robot control systems. In one example embodiment, the surgical robot control system includes a housing. A controller is located within the housing and is coupled to a socket. The socket receives a handheld surgical user interface therein to control a surgical instrument. The surgical instrument is connected to the surgical robot and includes an end effector and a mechanical interface to manipulate the end effector. The mechanical interface is coupled to the controller. At least one sensor is coupled to the controller and the socket to convert movement of the handheld surgical user interface into electrical signals corresponding to the movement of the surgical instrument. At least one feedback device is coupled to the controller to provide feedback to a user. The feedback is associated with a predetermined function of the surgical instrument.

A document (US2011/0295269A1) discloses a surgical cutting and fastening instrument that is motorized and configured for operation in connection with a robotic system. The instrument includes in one embodiment a charge accumulator device. separate from a battery, that provides additional power to the motor under certain conditions. In addition, the motor may include multiple windings.

A document (US5605272A) discloses a linear surgical stapler having clamping and firing triggers pivotally mounted to the frame of the instrument for pivotal rotation toward a hand grip. The clamping trigger is positioned between the firing trigger and the hand grip. Each of the triggers are pivotally mounted to pivot pins spaced from each other within the body portion of the frame. Pivotal rotation of the first trigger is independent of pivotal rotation of the second trigger. When the clamping trigger is pivotally rotated toward the hand grip, the clamping and firing transmission assemblies move distally in tandem. Before the first trigger is pivoted, the firing transmission assembly blocks pivotal rotation of the second trigger. When the first trigger is pivoted, the initial distal movement of the firing transmission assembly enables the firing trigger to be in a position to pivotally rotate, and therefore cause the firing of staples. Additionally, a feature enabling the retention of the clamping trigger in intermediate and fully clamped positions is disclosed, where upon release of the retention feature, the clamping trigger can return to its original position by overriding the intermediate position. Finally, although the invention is described with particular reference to a linear stapler containing clamping and firing triggers, it has broader applicability to any surgical instrument which has first and second triggers pivotally actuated to transmit work to an end effector for performing first and second surgical functions during a desired surgical procedure.

### Summary

The invention is as defined in the appendend claims. The disclosure provides an actuation mechanism and an ultrasonic surgical instrument to facilitate a surgeon to feel strength of a clamping force of jaws and to improve cutting effects of the ultrasonic surgical instrument.

An embodiment of the disclosure provides an ultrasonic surgical instrument. The ultrasonic surgical instrument includes an elongated body, a clamping assembly and an actuation mechanism configured to actuate the clamping assembly. The actuation mechanism includes: a handle portion; a first trigger and a second trigger pivotally engaged with the handle portion, respectively; a driving member configured to actuate the clamping assembly, wherein the clamping assembly is arranged on a distal portion of the elongated body; wherein the first trigger is engaged with the driving member through a first link, and the second trigger is engaged with the driving member through the first link and a second link, and wherein the first trigger is movable independently with respect to the second trigger during a portion of movement of the first trigger; wherein the handle portion (1) includes a stationary handle (12), and the first trigger (4) and the second trigger (5) are arranged on opposing sides of the stationary handle (12); and the actuation mechanism further includes a biasing member (6) configured for biasing the second trigger (5) until force against the driving member (9) exceeding a predetermined limit; wherein the force is generated by the movement of the first trigger.

In an implementation, movement of the first trigger depends on movement of the second trigger during a portion of movement thereof.

In an implementation, the second link pivotally coupled with the second trigger is engaged with the first link through a sliding member.

In an implementation, an obround hole is arranged on the first trigger for receiving a pivot coupled to the first link and the second link sliding within.

In an implementation, the second link pivotally coupled with the second trigger is engaged with the first link through a third link.

In an implementation, the second link pivotally coupled with the second trigger is engaged with the first link through a cam mechanism.

In an implementation, the second trigger is arranged in the handle portion and the biasing member is arranged in a body of the second trigger.

In an implementation, an angle formed between the first link and the second link is less than 180°.

In an implementation, an angle formed between the first link and the second link changes with movement of the second trigger.

In an implementation, during the portion of movement of the first trigger, the second trigger rotates in a direction opposite to a rotation direction of the first trigger for providing feedback to an operator of force applied on the driving member.

In an implementation, during the portion of movement of the first trigger, the second trigger rotates in a direction opposite to a rotation direction of the first trigger.

In an implementation, during the portion of movement of the first trigger, the second trigger rotates in a same direction as a rotation direction of the first trigger.

An actuation mechanism according to any one of the above-mentioned embodiments includes the mechanical advantage mechanisms configured to feedback the clamping force of the jaws and the second trigger configured to feedback the clamping force of the jaws to the operator. Thus during operation, a clamping force of the jaws may be fed back to the operator directly and accurately via the second trigger 5. In this way, the operator could sense the strength of the clamping force of the jaws and could adjust the clamping force of the jaws according to the strength of the feedback force conveniently, avoiding possible damages to tissue of a patient due to excessive clamping force. Additionally, through the arrangement of the double triggers, the operator may adjust the strength of the clamping force of the clamping assembly 3 according to actual conditions of the clamped tissue or vessel, thus expanding the application range of the ultrasonic surgical instrument.

### Brief Description of the Drawings

FIG. 1 illustrates an ultrasonic surgical instrument according to an embodiment of the disclosure.
FIG. 2 illustrates a structure of the ultrasonic surgical instrument according to the embodiment of the disclosure.
FIG. 3 illustrates the ultrasonic surgical instrument in a free state according to the embodiment of the disclosure.
FIG. 4 illustrates the ultrasonic surgical instrument with a closed clamping assembly according to the embodiment of the disclosure.
FIG. 5 illustrates the ultrasonic surgical instrument after a second trigger has moved according to the embodiment of the disclosure.
FIG. 6 illustrates an ultrasonic surgical instrument in a free state according to another embodiment of the present disclosure.
FIG. 7 illustrates the ultrasonic surgical instrument with a closed clamping assembly according to the embodiment of the disclosure.
FIG. 8 illustrates the ultrasonic surgical instrument after a second trigger has moved according to the embodiment of the disclosure.
FIG. 9 illustrates an ultrasonic surgical instrument in a free state according to another embodiment of the disclosure.
FIG. 10 illustrates the ultrasonic surgical instrument with a closed clamping assembly according to the embodiment of the disclosure.
FIG. 11 illustrates the ultrasonic surgical instrument after a second trigger has moved according to the embodiment of the disclosure.

### Reference numerals:

1-handle portion, 2-elongated body 2, 3-clamping assembly, 4-first trigger, 5-second trigger, 6-biasing member, 7-second link, 8-first link, 9-driving member, 10-pivot, 11-handle body, 12-handle, 13-third link, 41-first pivot, 42-obround hole, 43-protrusion, 51-second pivot.

### Detailed Description

Examples of the disclosure are described below in detail with reference to the drawings. The term "distal" used herein refers to a portion/part of an instrument that is farther away from an operator (e.g., a surgeon) and the term "proximal" refers to a portion/part of the instrument that is closer to the operator (e.g., the surgeon). It should be understood that the embodiments described herein are only used for describing and explaining the present disclosure, not for limiting the disclosure.

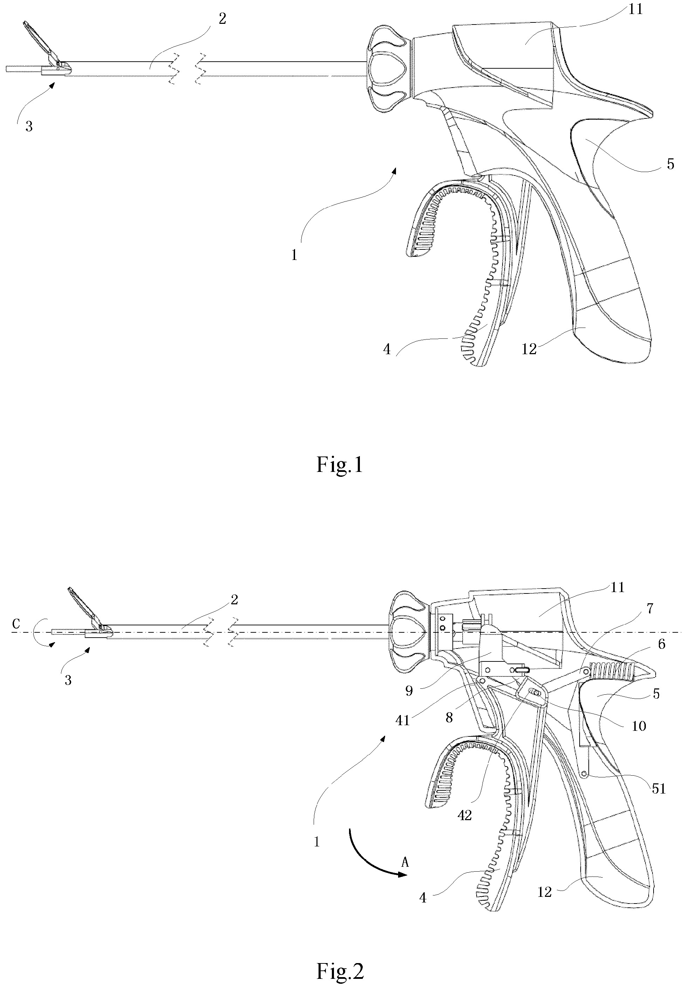

The disclosure provides an actuation mechanism for performing action and an ultrasonic surgical instrument. As illustrated by FIGs.2-5, the actuation mechanism includes a first trigger assembly and a second trigger assembly, both of which are movable. The first trigger assembly is provided with a first trigger 4, and the second trigger assembly is provided with a second trigger 5, where the first trigger 4 is movable independently with respect to the second trigger 5 during a portion of movement of the first trigger 4 and/or the second trigger 5.The actuation mechanism further includes a driving member 9 that is engaged with the first trigger assembly and the second trigger assembly, respectively. The first trigger assembly and the second trigger assembly are applied different mechanical advantages.

Specifically, the first trigger assembly includes the first trigger 4 and a first mechanical advantage mechanism, and the second trigger assembly includes the second trigger 5 and a second mechanical advantage mechanism.

According to the embodiment of the present disclosure, force applied onto the first trigger 4 and to the second trigger 5 may be delivered to the driving member 9 through the rotatable first and second triggers 4, 5 and the first and second mechanical advantage mechanisms, respectively. Specifically, the first mechanical advantage mechanism is engaged with the first trigger 4, adapted for delivering the force applied onto the first trigger 4 to the driving member 9. The second mechanical advantage mechanism is engaged with the second trigger 5, adapted for delivering the force applied onto the second trigger 5 to the driving member 9, so as to amplify force delivered to the driving member 9 when less force is applied onto the first trigger 4 and the second trigger 5, respectively, which amplifies output force of the entire actuation mechanism. It should be noted that the mechanical advantage mechanism is not limited to amplifying force from the first trigger 4 or the second trigger 5. It can also be designed for diminishing the force or only equivalently transmitting the force based on various conditions.

Furthermore, the mechanical advantage of the second trigger assembly is determined by a position of the first trigger 4. The term "the mechanical advantage" used herein refers to a ratio of output force to input force of a mechanical system. Taking a lever system as example, which may be one of the most common mechanical advantage mechanisms, the mechanical advantage thereof refers to a ratio of the input force applied on one end to the output force from the other end of the bar. Structures according to embodiments of the present disclosure are illustrated in detail hereinafter.

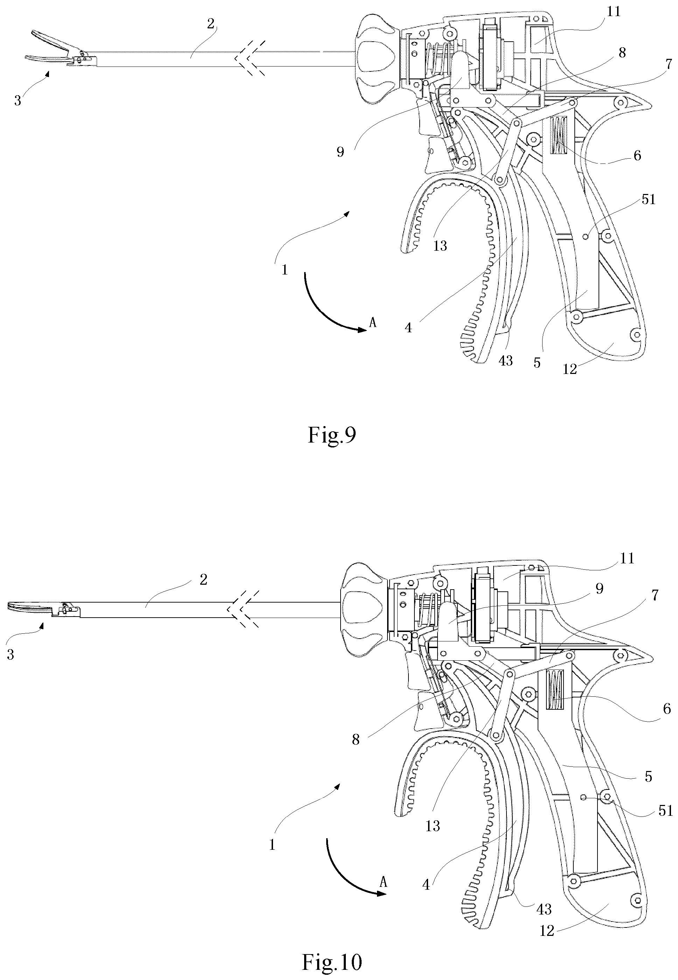

As illustrated by FIG.1, one embodiment of the present disclosure provides an ultrasonic surgical instrument, which includes a handle portion 1, an elongated body 2 and a clamping assembly 3. The handle portion 1 includes a handle body 11 and a stationary handle 12. The clamping assembly 3 and the elongated body 2 can rotate about a longitudinal axis C of the instrument with respect to the handle portion 1. The positions of the first and second triggers 4, 5 can be arranged according to various requirements. For example, as illustrated by FIGs. 2-8, the first trigger 4 and the second trigger 5 are arranged on opposing sides of the handle portion 1, respectively. Specifically, the first trigger 4 and the second trigger 5 are arranged on opposing side of the stationary handle 12 of the handle portion 1, and may be contacted by an operator grasping the stationary handle 12. For example, the first trigger 4 and the second trigger 5 can be contacted by four fingers (except for thumb) of a hand and a portion between thumb and index finger, respectively. Alternatively, the first trigger 4 is arranged on one side of the stationary handle 12, while the second trigger 5 is arranged inside the handle 12. As illustrated by FIGs. 9-11, when the operator grasps the instrument, only the first trigger 4 is contactable by his/her hand.

By using different mechanical advantage mechanisms, force applied on the first trigger 4 and the second trigger 5 may be amplified to different values, respectively, where mechanical advantage of the second trigger assembly may be determined or controlled by the position of the first trigger assembly. Specifically, as illustrated by FIG.2, the actuation mechanism further includes a pivot 10, a first link 8 and a second link 7. The first link 8 is engaged with the second link 7 via the pivot 10. Further, the distal end of the first link 8 is engaged with a driving member 9 that slidably arranged in the ultrasonic surgical instrument. The proximal end of the second link 7 is engaged with the second trigger 5. The pivot 10 is engaged with the first trigger 4 so as to be pushed by the first trigger 4 during rotation thereof, and the driving member 9 may be pushed by the first link 8 and/or the second trigger 5 may be pushed by the second link 7. Alternatively, the second link 7 may be pushed by the second trigger 5 during the rotation thereof, and the driving member 9 may be pushed by the first link 8.

The driving member 9 is configured to actuate the clamping assembly 3 that is arranged at the distal portion of the ultrasonic surgical instrument to open or close through the elongated body 2. Specifically, a sliding member, for example, a sliding track, is arranged in the handle body 11, and the driving member 9 is assembled onto the sliding track. As illustrated by FIGs.2, 3 and 4, the driving member 9 may reciprocate along the slide track. The clamping assembly 3 may be actuated to close when the driving member 9 moves distally, whereas the clamping assembly 3 may be actuated to open when the driving member 9 moves proximately. Specifically, the elongated body 2 includes an inner tube and an outer tube, through which the clamping assembly 3 is driven to open or close by the driving member 9.

In another implementation, the actuation mechanism according to the embodiment of the disclosure further includes a biasing member 6 to limit rotation of the second trigger 5. Specifically, the biasing member 6 prevents the second link 7 from moving proximately so as to prevent movement of the second trigger 5 until force applied on the driving member 9 exceeds a predetermined limit. In other words, when the first trigger 4 rotates in a direction indicated by an arrow A to actuate the pivot 10 to move upwards, the movement of the pivot 10 may provide the second link 7 with a tendency of moving proximately; then the biasing member 6 may provide the second trigger 5 with a resistance for eliminating the force delivered thereto, so as to further prevent the second trigger 5 from rotating and keep the second trigger 5 stationary. The second trigger 5 may stay stationary until the force delivered thereto exceeds the resistance limit of the biasing member 6. Specifically, the biasing member 6 can be any elastic member that is capable of providing resistance/reverse force, such as a compression spring, a tension spring, or a torsion spring. The arrangement of the biasing member 6 may be designed variously, according to specific deformation modes of the compression spring, the tension spring, or the torsion spring, and etc., as long as a certain reverse force can be provided accordingly during force delivery from the second link 7 to the second trigger 5.For example, a compression spring is arranged in the handle body 11, with two ends being engaged with the proximal inner wall of the handle body 11 and the proximal end of the second link 7, respectively. When force is delivered to the second link 7, a resistance may be provided by the compression spring to limit motion of the second trigger 5.

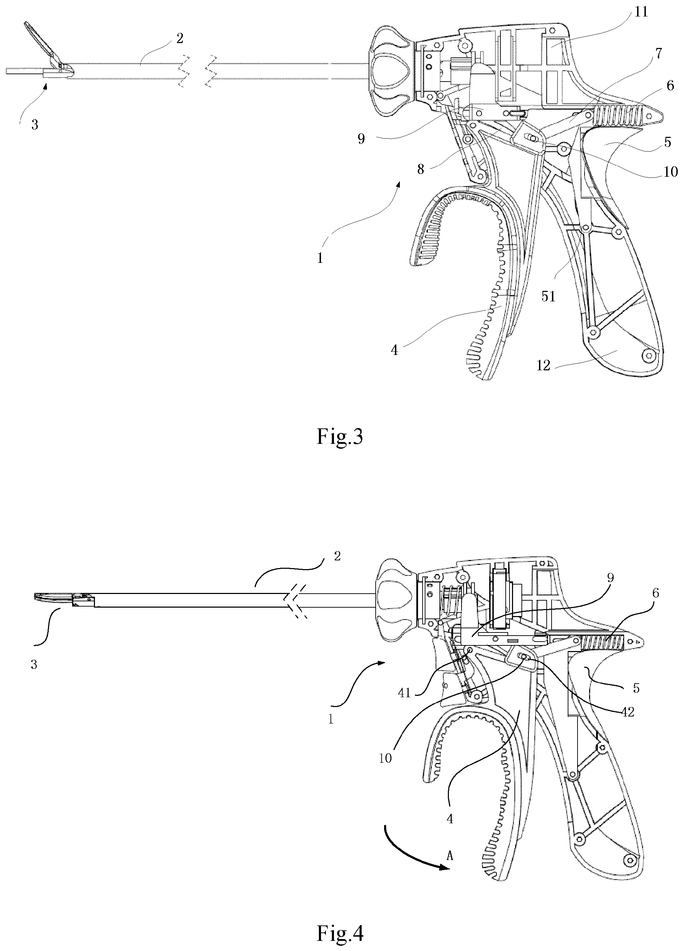

Hereinafter, engagement and movement of the actuation mechanism is described in detail in combination with various states of the ultrasonic surgical instrument according to the embodiment of the disclosure. Firstly, FIG. 3 illustrates the ultrasonic surgical instrument in a free state, where the clamping assembly 3 is open and the actuation mechanism is in a free state and under no force(s). As illustrated by FIG.4, when the first trigger 4 is pulled in a direction indicated by an arrow A, the first trigger 4 pivots about a first pivot 41 and actuates the pivot 10 to move upwards, where an angle formed between the first link 8 and the second link 7 increases. Since the second link 7 is limited by the biasing member 6 from moving proximately, the pivot 10 may push the first link 8 to move distally during upward movement thereof, so as to further actuate the driving member 9 to move to distally for closing the clamping assembly 3.

FIG.4 shows a state of the ultrasonic surgical instrument according to one embodiment of the present disclosure, where the clamping assembly 3 is fully closed. It can be seen from FIG.3 and FIG.4 that the second trigger 5 is prevented from further movement by the biasing member 6, e.g., the compression spring, so the driving member 9 is pushed distally by the first link 8 for closing the clamping assembly 3.

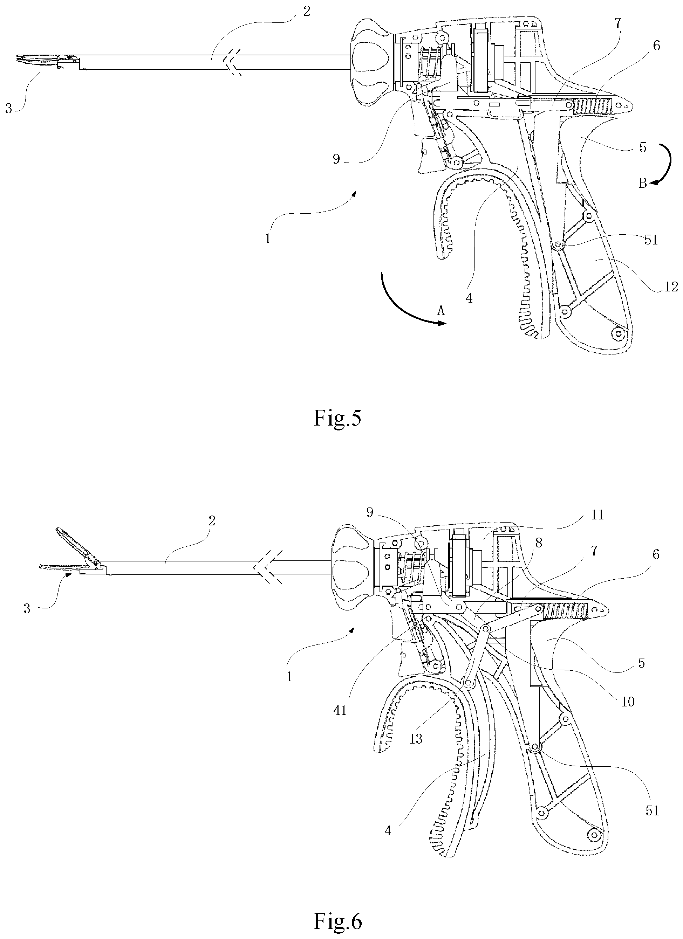

After the clamping assembly is closed, as shown by FIG.5, the first trigger 4 is further pulled in the direction indicated by the arrow A. The second trigger 5 may rotate about a second pivot 51 in a direction indicated by an arrow B illustrated by FIG.5 when force delivered to the second link 7 through the driving member 9, the pivot 10 and the first trigger 4 exceeds the resistance limit of the biasing member 6. Thus, the operator may receive a feedback of force from the second trigger 5 through the part between thumb and index finger of his/her hand, so as to further get to know the clamping force applied on the clamping assembly. Moreover, the operator could further apply force to the second trigger 5 through the part between the thumb and the index finger of his/her hand, which may further be delivered to the first link 8 and the driving member 9 so as to apply an additional clamping force on the closed clamping assembly 3 through the second trigger 5, the second link 7 and the pivot 10. By pressing the first trigger 4 and the second trigger 5 simultaneously, net force of the force from the two triggers can be delivered to the clamping assembly 3 by the actuation mechanism according to the embodiment, which means the clamping force of the clamping assembly 3 is increased. Therefore, comparing with a conventional ultrasonic surgical instrument having only one trigger, by using the actuation mechanism according to the embodiment, the operator can press two triggers to increase the force acting upon the clamping assembly 3, so that net force could be applied to the clamping assembly 3, which makes the ultrasonic surgical instrument available for a wider range of applications.

Specifically, the angle formed between the first link 8 and the second link 7 is less than 180°. During rotation of the first trigger 4 and/or of the second trigger 5, the angle between the first link 8 and the second link 7 increases gradually but under 180°. In other words, when the first trigger 4 and the second trigger 5 move to their limited positions, respectively, the angle between the first link 8 and the second link 7 is less than 180°. In an implementation, when the first trigger 4 and the second trigger 5 are under no force, the angle formed between the first link 8 and the second link 7 is within a range from 60° to 150°. For example, the angle could have any value between 60° and 150° such as 60°, 70°, 80°, 90°, 100°, 110°, 120°, 130°, 140°, or 150°.When the first trigger 4 and the second trigger 5 are in their limited positions, respectively, the angle between the first link 8 and the second link 7 is less than 180°, for example, 175° or 170°.

In an implementation, the first link 8 is shorter than that of the second link 7, so that more horizontal components of force applied on the first trigger 4 and the second trigger 5 can be delivered to the first link 8 for actuating the driving member 9 to move distally.

In an alternative embodiment, the actuation mechanism further includes an off-center member, through which the mechanical advantage mechanism is actuated by the first trigger 4. Specifically, the off-center member is arranged on the first trigger 4, which may be actuated to rotate through rotation of the first trigger 4, so as to actuate the mechanical advantage mechanism to move.

Furthermore, for improving ergonomic effects of the instrument, in another embodiment, a plurality of grooves are arranged on the first trigger 4 for pairing with the fingers of the operator, and an arc portion is provided for the second trigger 5, for pairing with the part of the hand between the thumb and the index finger of the operator, such that the operator may apply force more comfortable through his/her hand. In an implementation, as illustrated by Fig.2, it is preferred three grooves are arranged on the first trigger.

To improve stability of the actuation mechanism, in one implementation, the first trigger assembly is engaged with the second trigger assembly through a sliding member. For example, an obround hole 42 is arranged in the first trigger 4, as illustrated by FIG. 2, and the pivot 10 is slidably disposed within the obround hole 42. Compared with Figs. 3 and 4, it can be seen that, when the first trigger is in the free state, the place where the pivot 10 locates within the obround hole 42 is different from that when it is under force, and the position where the pivot 10 contacts with the first trigger 4 changes as the first trigger 4 and the second trigger 5 rotate. However, no matter how it changes, the pivot 10 remains within the obround hole 42, maintaining engagement with the first trigger 4 when the first trigger 4 is forced to rotate as well as when the first trigger 4 is under no force. Moreover, a recovering spring may also be arranged in the first trigger 4 (configured for forcing the first trigger 4 to recover to its initial position), such as a torsion spring, which is arranged for forcing the first link 8 and the second link 7 returning to their initial positions through the orbound hole 42.

In another embodiment, as illustrated by FIG. 6, one end of a third link 13 is engaged with the first trigger 4 and the other end thereof is engaged with one end of the first link 8 and with one end of the second link 7, respectively. The other end of the second link 7 is engaged with the second trigger 5 and the other end of the first link 8 is engaged with the driving member 9. In one implementation, a sliding member, e.g., a sliding track, is arranged in the handle body 11, and for example, the driving member 9 is slidably assembled with the sliding track. As illustrated by FIG.2, FIG.3 and FIG.4, the driving member 9 may reciprocate along the slide track. The clamping assembly 3 may be actuated to close when the driving member 9 slides distally, and to open when the driving member 9 slides proximately. When the first trigger 4 rotates about the first pivot when being pulled in the direction indicated by an arrow A as illustrated by Fig. 6, the third link 13 is forced to move so as to actuate movement of the first link 8 and the driving member 9. In one implementation, a biasing member 6, such as, a compression spring, may be provided in the actuation mechanism according to the embodiment of the disclosure. The biasing member 6 is such arranged that one end thereof presses against the inner wall of the proximate portion of the handle body 11 and the other end presses against the proximal end of the second link 7. The function of the biasing member 6 is substantially similar to the function of the biasing member according to the previous embodiment, and thus details are omitted here.

Similarly, engagement and movement of the actuation mechanism according to the above mentioned embodiments are described in detail with reference to the drawings the different states of the ultrasonic surgical instrument according to the above-mentioned embodiments of the present disclosure.

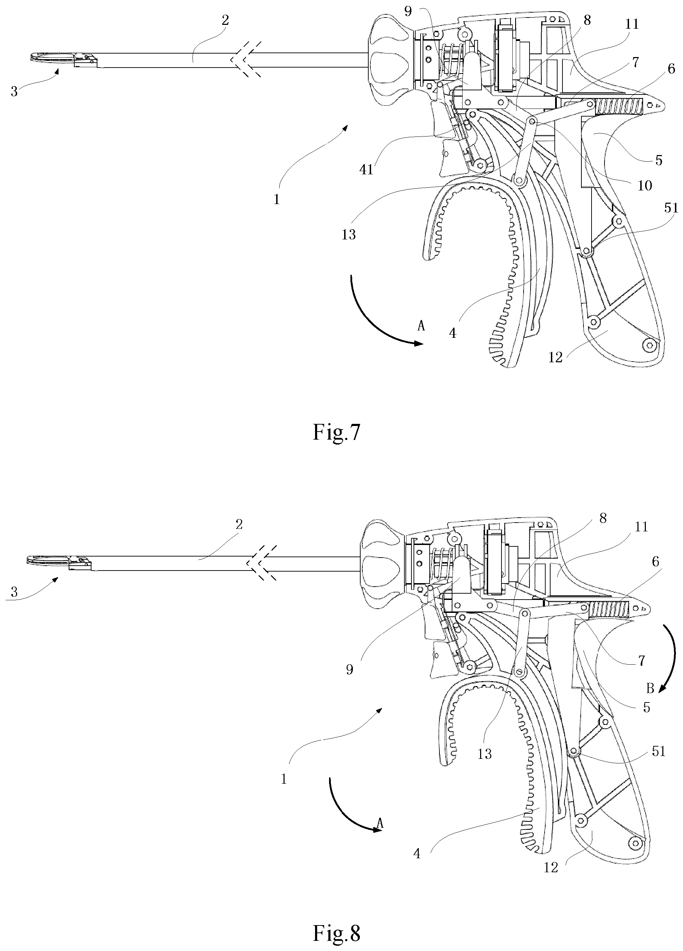

Firstly, FIG. 6 shows the ultrasonic surgical instrument according to the embodiment of the present disclosure in the free state. The actuation mechanism includes the first link 8, the second link 7 and the third link 13. One end of the first link 8, one end of the second link 7 and one end of the third link 13 are engaged. The other end of the first link 8 is engaged with a sliding member, i.e., a sliding block engaged with the driving member 9 and assembled on the sliding track. The proximal end of the second link 8 is engaged with the second trigger 5 and the other end of the third link 13 is engaged with the first trigger 4. The third link 13 is actuated to move based on movement of the first trigger 4, as illustrated by FIGs. 6 and 7, the three links are such arranged to form as a letter Y.

The clamping assembly 3 that is disposed at the distal portion of the ultrasonic surgical instrument may be closed when the first trigger 4 is pulled in the direction indicated by the arrow A, as illustrated by FIG. 7. Since the force delivered to the second trigger 5 through the second link 7 does not exceed the resistance limit of the biasing member 6, the second trigger 5 does not move accordingly. Then the first trigger 4 forces the third link 13 to move upwards and pushes the first link 8 to move distally, so that the driving member 9 is forced to move distally for closing the clamping assembly 3.

Further pulling the first trigger 4, as illustrated by FIG. 8, when the force delivered to the second trigger 5 through the second link 7 exceeds a predetermined resistance limit of the biasing member 6,, the biasing member 6 is compressed so that the second link 7 forces the second trigger 5 to pivot about the second pivot 51 in the direction indicated by an arrow B. Then force applied on the second trigger 5 may be delivered back to the operator to the part of his/her hand between the thumb and index finger, through which, the operator may be aware of the clamping force of the clamping assembly accordingly. Moreover, the operator could apply additional force to the second trigger 5, for example, through the part of his/her hand between the thumb and the index finger, and the force applied on the second trigger 5 may be further delivered to the driving member 9 through the second link 7 and the first link 8, and ultimately be applied to the clamping assembly for increasing the clamping force. By pressing the first trigger 4 and the second trigger 5 simultaneously, net force of the force of the two triggers can be delivered to the clamping assembly 3 through the actuation mechanism according to the embodiment, increasing the clamping force of the clamping assembly 3.

In another implementation, the first link 8, the second link 7 and the third link 13 of the above described embodiment may be considered as an off-center mechanism. That is, during the upward movement of the pivot 10, it may move beyond the straight line formed by the first and second links 8 and 7. In other words, during the motion of the entire actuation mechanism, the angle formed between the first and second links 8 and 7 increases gradually, and may exceed 180°.

According to another embodiment as illustrated by FIGs. 9, 10 and 11, the structure of another actuation mechanism is substantially similar to the structure of the actuation mechanism according to the above-mentioned embodiment, except for the arrangement of the second trigger 5. To ease description, the common elements of the structures are omitted herein and only the second trigger 5 of the actuation mechanism according to this embodiment of the disclosure is described in detail. It can be seen from FIG.9 that the second trigger 5 is arranged inside the handle body 11, and is provided with a groove for receiving the biasing member 6, e.g., the compression spring. A stop (not shown) is arranged on the handle body 11. One end of the biasing member 6 presses against the stop on the handle body 11 and the other end thereof presses against an inner wall of the groove of the second trigger 5.

FIG. 9 illustrates the ultrasonic surgical instrument according to the above described embodiment in the free state. When the first trigger 4 is pulled in a direction indicated by an arrow A, the clamping assembly 3 at the distal portion of the ultrasonic surgical instrument is closed accordingly, as illustrated by FIG. 10. Since the force delivered to the second trigger 5 from the second link 7 has not exceeded the resistance limit of the biasing member 6, the second trigger 5 does not move as the first trigger 5 moves. Then the first trigger 4 forces the third link 13 to move upwards and pushes the first link 8 to move distally, so that the driving member 9 is forced to move distally for closing the clamping assembly 3.

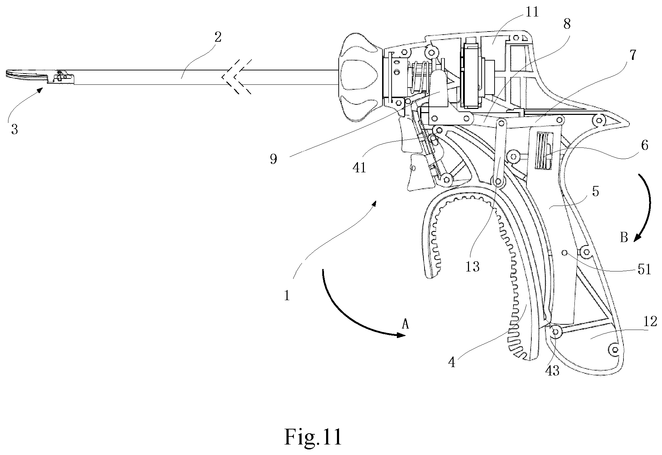

After the first trigger 4 is further pulled, as illustrated by FIG. 11, the biasing member 6 may be compressed when the force applied to the second trigger 5 through the second link 7 exceeds the resistance limit of the biasing member 6, so that the second trigger 5 is driven to rotate about the second pivot 51 in a direction indicated by an arrow B by the second link 7. As illustrated by FIGs. 9, 10 and 11, the first trigger 4 further includes a protrusion 43, when the protrusion 43 on the first trigger 4 presses against the second trigger 5, the first trigger 4 is further pulled, the second trigger 5 is driven to rotate counterclockwise about the second pivot 51 by the protrusion 43, such that, the driving member 9 may be further pushed to move distally through the second link 7 and the first link 8 to apply an additional force to the closed clamping assembly 3 for increasing the clamping force of the clamp assembly.

According to another embodiment, based on the above-mentioned embodiment, the second trigger 5 is engaged with the first link 8 through a cam mechanism, that is, the first trigger 4 is engaged with the second link 7 through the cam mechanism. Specifically, the cam mechanism is rotated by rotation of the first trigger 4, and the first link 8 is moved by rotation of the cam mechanism so as to actuate the driving member 9 to move.

Moreover, in the above mentioned embodiments, the mechanical advantage mechanism and the second trigger 5 are adapted for providing feedback of clamping force to the operator. Thus during operation, a clamping force of the clamp assembly may be delivered back to the operator directly and accurately via the second trigger 5, which may help the operator be aware of the clamping force applied on the clamping assembly and make adjustment accordingly , avoiding potential damages to tissue caused by excessive clamping force. Additionally, through the arrangement of the double triggers, the operator may adjust the clamping force of the clamping assembly 3 according to actual conditions of the clamped tissue or vessel of a patient, which may expand the application range of the ultrasonic surgical instrument.

Evidently those skilled in the art can make various modifications and variations to the present disclosure without departing from the scope of the present disclosure. Thus the present disclosure is also intended to encompass these modifications and variations thereto so long as the modifications and variations come into the scope of the claims appended to the present disclosure and their equivalents.

## Claims

1. An ultrasonic surgical instrument comprising an elongated body (2), a clamping assembly (3) and an actuation mechanism configured to actuate the clamping assembly (3), **characterized in that**, the actuation mechanism comprises:
a handle portion (1);
a first trigger (4) and a second trigger (5) pivotally engaged with the handle portion (1), respectively;
a driving member (9) configured to actuate the clamping assembly (3), wherein the clamping assembly (3) is arranged on a distal portion of the elongated body (2);
wherein the first trigger (4) is engaged with the driving member (9) through a first link (8), and the second trigger (5) is engaged with the driving member (9) through the first link (8) and a second link (7), and wherein the first trigger (4) is movable independently with respect to the second trigger (5) during a portion of movement of the first trigger (4);
wherein the handle portion (1) comprises a stationary handle (12), and the first trigger (4) and the second trigger (5) are arranged on opposing sides of the stationary handle (12);
wherein the actuation mechanism further comprises a biasing member (6) configured for biasing the second trigger (5) until force against the driving member (9) exceeding a predetermined limit; wherein the force is generated by the movement of the first trigger (4).

2. The ultrasonic surgical instrument according to claim 1, wherein movement of the first trigger (4) depends on movement of the second trigger (5) during a portion of movement thereof.

3. The ultrasonic surgical instrument according to claim 1 or 2, wherein the second link (7) pivotally coupled with the second trigger (5) is engaged with the first link (8) through a sliding member.

4. The ultrasonic surgical instrument according to claim 1 or 2, wherein an obround hole (42) is arranged on the first trigger (4) for receiving a pivot (10) coupled to the first link (8) and the second link (7) sliding within.

5. The ultrasonic surgical instrument according to claim 1 or 2, wherein the second link (7) pivotally coupled with the second trigger (5) is engaged with the first link (8) through a third link (13).

6. The ultrasonic surgical instrument according to claim 1 or 2, wherein the second link (7) pivotally coupled with the second trigger (5) is engaged with the first link (8) through a cam mechanism.

7. The ultrasonic surgical instrument according to of claim 1, wherein the second trigger (5) is arranged in the handle portion (1) and the biasing member (6) is arranged in a body of the second trigger (5).

8. The ultrasonic surgical instrument according to any one of claims 1-7, wherein an angle formed between the first link (8) and the second link (7) is less than 180°.

9. The ultrasonic surgical instrument according to any one of claims 1-8, wherein an angle formed between the first link (8) and the second link (7) changes with movement of the second trigger (5).

10. The ultrasonic surgical instrument according to any one of claims 1-6 or 8-9, wherein during the portion of movement of the first trigger (4), the second trigger (5) rotates in a direction opposite to a rotation direction of the first trigger (4) for providing feedback to an operator of force applied on the driving member (9).

11. The ultrasonic surgical instrument according to claim 7, wherein during the portion of movement of the first trigger (4), the second trigger (5) rotates in a direction opposite to a rotation direction of the first trigger (4).

12. The ultrasonic surgical instrument according to any one of claims 1-11, wherein during the portion of movement of the first trigger (4), the second trigger (5) rotates in a same direction as a rotation direction of the first trigger (4).

## Patentansprüche

1. Chirurgisches Ultraschallinstrument, das einen länglichen Körper (2), eine Klemmanordnung (3) und einen Betätigungsmechanismus umfasst, der zum Betätigen der Klemmanordnung (3) konfiguriert ist, **dadurch gekennzeichnet, dass** der Betätigungsmechanismus umfasst:
ein Griffteil (1);
einen ersten Auslöser (4) und einen zweiten Auslöser (5), die jeweils schwenkbar mit dem Griffteil (1) verbunden sind;
ein Antriebselement (9), das so konfiguriert ist, dass es die Klemmanordnung (3) betätigt, wobei die Klemmanordnung (3) an einem distalen Abschnitt des länglichen Körpers (2) angeordnet ist;
wobei der erste Auslöser (4) über ein erstes Verbindungsglied (8) mit dem Antriebselement (9) in Eingriff steht und der zweite Auslöser (5) über das erste Verbindungsglied (8) und ein zweites Verbindungsglied (7) mit dem Antriebselement (9) in Eingriff steht, und wobei der erste Auslöser (4) während eines Teils der Bewegung des ersten Auslösers (4) unabhängig in Bezug auf den zweiten Auslöser (5) beweglich ist;
wobei der Griffteil (1) einen stationären Griff (12) umfasst und der erste Auslöser (4) und der zweite Auslöser (5) auf gegenüberliegenden Seiten des stationären Griffs (12) angeordnet sind;
wobei der Betätigungsmechanismus ferner ein Vorspannelement (6) umfasst, das so konfiguriert ist, dass es den zweiten Auslöser (5) vorspannt, bis die Kraft gegen das Antriebselement (9) einen vorgegebenen Grenzwert überschreitet; wobei die Kraft durch die Bewegung des ersten Auslösers (4) erzeugt wird.

2. Chirurgisches Ultraschallinstrument nach Anspruch 1, wobei die Bewegung des ersten Auslösers (4) von der Bewegung des zweiten Auslösers (5) während eines Teils der Bewegung desselben abhängt.

3. Chirurgisches Ultraschallinstrument nach Anspruch 1 oder 2, wobei das zweite Verbindungsglied (7), das schwenkbar mit dem zweiten Auslöser (5) gekoppelt ist, mit dem ersten Verbindungsglied (8) durch ein Gleitelement in Eingriff steht.

4. Chirurgisches Ultraschallinstrument nach Anspruch 1 oder 2, wobei am ersten Auslöser (4) ein ovales Loch (42) zur Aufnahme eines Drehzapfens (10) angeordnet ist, der mit dem ersten Verbindungsglied (8) und dem darin gleitenden zweiten Verbindungsglied (7) verbunden ist.

5. Chirurgisches Ultraschallinstrument nach Anspruch 1 oder 2, wobei das Verbindungsglied (7), das schwenkbar mit dem zweiten Auslöser (5) verbunden ist, über ein drittes Verbindungsglied (13) mit dem ersten Verbindungsglied (8) in Eingriff steht.

6. Chirurgisches Ultraschallinstrument nach Anspruch 1 oder 2, wobei das zweite Verbindungsglied (7), das schwenkbar mit dem zweiten Auslöser (5) gekoppelt ist, über einen Nockenmechanismus mit dem ersten Verbindungsglied (8) in Eingriff steht.

7. Chirurgisches Ultraschallinstrument nach Anspruch 1, wobei der zweite Auslöser (5) im Griffteil (1) angeordnet ist und das Vorspannelement (6) in einem Körper des zweiten Auslösers (5) angeordnet ist.

8. Chirurgisches Ultraschallinstrument nach einem der Ansprüche 1 bis 7, wobei ein zwischen dem ersten Verbindungsglied (8) und dem zweiten Verbindungsglied (7) gebildeter Winkel weniger als 180° beträgt.

9. Chirurgisches Ultraschallinstrument nach einem der Ansprüche 1 bis 8, wobei sich ein zwischen dem ersten Verbindungsglied (8) und dem zweiten Verbindungsglied (7) gebildeter Winkel mit der Bewegung des zweiten Auslösers (5) ändert.

10. Chirurgisches Ultraschallinstrument nach einem der Ansprüche 1 bis 6 oder 8 bis 9, wobei sich der zweite Auslöser (5) während des Bewegungsabschnitts des ersten Auslösers (4) in einer Richtung dreht, die einer Drehrichtung des ersten Auslösers (4) entgegengesetzt ist, um einem Bediener eine Rückmeldung über die auf das Antriebselement (9) ausgeübte Kraft zu geben.

11. Chirurgisches Ultraschallinstrument nach Anspruch 7, wobei sich der zweite Auslöser (5) während des Bewegungsabschnitts des ersten Auslösers (4) in einer Richtung dreht, die einer Drehrichtung des ersten Auslösers (4) entgegengesetzt ist.

12. Chirurgisches Ultraschallinstrument nach einem der Ansprüche 1 bis 11, wobei sich der zweite Auslöser (5) während des Bewegungsabschnitts des ersten Auslösers (4) in derselben Richtung wie eine Drehrichtung des ersten Auslösers (4) dreht.

## Revendications

1. Instrument chirurgical à ultrasons comprenant un corps allongé (2), un ensemble de serrage (3) et un mécanisme d'actionnement configuré pour actionner l'ensemble de serrage (3), **caractérisé en ce que** le mécanisme d'actionnement comprend :
une partie poignée (1) ;
une première gâchette (4) et une seconde gâchette (5) engagées de manière pivotante avec la partie poignée (1), respectivement ;
un élément d'entraînement (9) configuré pour actionner l'ensemble de serrage (3), l'ensemble de serrage (3) étant disposé sur une partie distale du corps allongé (2) ;
dans lequel la première gâchette (4) est engagée avec l'élément d'entraînement (9) par l'intermédiaire d'une première liaison (8), et la seconde gâchette (5) est engagée avec l'élément d'entraînement (9) par l'intermédiaire de la première liaison (8) et d'une seconde liaison (7), et dans lequel la première gâchette (4) est mobile indépendamment par rapport à la seconde gâchette (5) pendant une partie du mouvement de la première gâchette (4) ;
dans lequel la partie poignée (1) comprend une poignée fixe (12), et la première gâchette (4) et la seconde gâchette (5) sont disposées sur les côtés opposés de la poignée fixe (12) ;
le mécanisme d'actionnement comprend en outre un élément de sollicitation (6) configuré pour solliciter la seconde gâchette (5) jusqu'à ce que la force exercée contre l'élément d'entraînement (9) dépasse une limite prédéterminée ; dans lequel la force est générée par le mouvement de la première gâchette (4).

2. Instrument chirurgical à ultrasons selon la revendication 1, dans lequel le mouvement de la première gâchette (4) dépend du mouvement de la seconde gâchette (5) pendant une partie de son mouvement.

3. Instrument chirurgical à ultrasons selon la revendication 1 ou 2, dans lequel la seconde liaison (7) couplé de manière pivotante à la seconde gâchette (5) est engagé avec la première liaison (8) par l'intermédiaire d'un élément coulissant.

4. Instrument chirurgical à ultrasons selon la revendication 1 ou 2, dans lequel un trou oblong (42) est disposé sur la première gâchette (4) pour recevoir un pivot (10) couplé à la première liaison (8) et à la seconde liaison (7) coulissant à l'intérieur.

5. Instrument chirurgical à ultrasons selon la revendication 1 ou 2, dans lequel la seconde liaison (7) couplé de manière pivotante à la seconde gâchette (5) est engagé avec la première liaison (8) par l'intermédiaire d'une troisième liaison (13).

6. Instrument chirurgical à ultrasons selon la revendication 1 ou 2, dans lequel la seconde liaison (7) couplé de manière pivotante à la seconde gâchette (5) est engagé avec la première liaison (8) par l'intermédiaire d'un mécanisme de came.

7. Instrument chirurgical à ultrasons selon la revendication 1, dans lequel la seconde gâchette (5) est disposée dans la partie de la poignée (1) et l'élément de sollicitation (6) est disposé dans un corps de la seconde gâchette (5).

8. Instrument chirurgical à ultrasons selon l'une des revendications 1 à 7, dans lequel un angle formé entre la première liaison (8) et la seconde liaison (7) est inférieur à 180°.

9. Instrument chirurgical à ultrasons selon l'une des revendications 1 à 8, dans lequel un angle formé entre la première liaison (8) et la seconde liaison (7) change avec le mouvement de la seconde gâchette (5).

10. Instrument chirurgical à ultrasons selon l'une des revendications 1 à 6 ou 8 à 9, dans lequel, pendant la partie du mouvement de la première gâchette (4), la seconde gâchette (5) tourne dans un sens opposé au sens de rotation de la première gâchette (4) afin de fournir à l'opérateur un retour d'information sur la force appliquée sur l'élément d'entraînement (9).

11. Instrument chirurgical à ultrasons selon la revendication 7, dans lequel, pendant la partie du mouvement de la première gâchette (4), la seconde gâchette (5) tourne dans un sens opposé au sens de rotation de la première gâchette (4).

12. Instrument chirurgical à ultrasons selon l'une des revendications 1 à 11, dans lequel, pendant la partie du mouvement de la première gâchette (4), la seconde gâchette (5) tourne dans le même sens que le sens de rotation de la première gâchette (4).
